Europäisches Patentamt

European Patent Office

Office européen des brevets

(11) Publication number: **0 365 347**
A1

## EUROPEAN PATENT APPLICATION

(21) Application number: 89310814.2

(22) Date of filing: 20.10.89

(51) Int. Cl.5: **C01F 5/14** , **C01F 5/20** , **C01F 5/22**

(30) Priority: 20.10.88 JP 264811/88

(43) Date of publication of application:
25.04.90 Bulletin 90/17

(84) Designated Contracting States:
DE ES FR GB IT NL

(71) Applicant: KYOWA CHEMICAL INDUSTRY CO.,
LTD.
305, Yashimanishi-machi
Takamatsu-shi Kagawa-ken(JP)

(72) Inventor: Miyata, Shigeo
251-1, Yashimanishi-machi
Takamatsu-shi Kagawa-ken(JP)
Inventor: Anabuki, Hitoshi
1389, Kawabe-machi
Takamatsu-shi Kagawa-ken(JP)
Inventor: Hirose, Toru
1273, Shido-machi Okawa-gun
Kagawa-ken(JP)

(74) Representative: Myerscough, Philip Boyd et al
J.A.Kemp & Co. 14, South Square Gray's Inn
London, WC1R 5EU(GB)

(54) High-activity and high-dispersibility magnesium hydroxide and process for the production thereof.

(57) The invention provides a magnesium hydroxide having a BET specific surface area of 20 to 50 $m^2/g$, a 50 percent diameter, as a secondary particle, of not more than 1.5 $\mu m$ and a 90 percent diameter, as a secondary particle, of not more than 4.0 $\mu m$, and a process for the production of a magnesium hydroxide which comprises heating a basic magnesium chloride or nitrate of the formula $Mg(OH)_{2-x}A_x \cdot mH_2O$ wherein A represents Cl or $NO_3$, x is in the range of $0<x<0.2$, and m represents a number of from 0 to 6, and having main spacings of 8.2Å, 4.1Å, 2.7Å, 2.0Å, 1.56Å and 1.54Å measured by X-ray diffraction, in a reaction mother liquor of the basic magnesium chloride or nitrate under atmospheric pressure or below at a temperature between 50°C and 120°C.

EP 0 365 347 A1

Xerox Copy Centre

**High-activity and high-dispersibility magnesium hydroxide and process for the production thereof**

Field of the Invention

The present invention relates to a high-activity and high-dispersibility magnesium hydroxide and a process for the production thereof. More specifically, it relates to a magnesium hydroxide having a BET specific surface area of 20 to 50 $m^2/g$ and a 50 percent diameter, as a secondary particle, of not more than 1.5 $\mu$m and a 90 percent diameter, as a secondary particle, of not more than 4.0 $\mu$m, and a process for the production thereof.

Prior Art of the Invention

Magnesium hydroxide is a basic substance, and due to its chemical property, it is used as a viscosity thickening agent for FRP, a neutralizing agent for an acidic substance in a resin, an antacid in a digestive medicine, or the like. These uses are based on utilization of basicity of magnesium hydroxide, i.e. its ability to neutralize acid. Hence, preferred is a magnesium hydroxide in which its BET specific surface area is larger, i.e. its crystallite diameter is smaller and the number of crystallites in their aggregate is smaller, i.e. the diamter of a secondary particle which is the aggregate of crystallites is smaller.

However, in conventional magnesium hydroxide, its BET specific surface area is in the range of from 20 to 50 $m^2/g$, but its 50 percent diameter as a secondary particle is about 3 to about 20 $\mu$m, and its 90 % percent diameter as a secondary particle is about 8 to about 40 $\mu$m. That is, since conventional high-activity magnesium hydroxide having a BET specific surface area of not less than 20 $m^2/g$ has poor dispersibility, it, in fact, does not exhibit any sufficient chemical activity which could be expected from its BET specific surface area.

For example, when conventional magnesium hydroxide is used as a viscosity thickening agent for FRP, there are problems such as a failure of a resin in impregnation into a glass fiber due to too high an initial viscosity thereof, degradation of productivity due to too low a viscosity thickening rate thereof, poor mechanical strength due to its low finally attainable viscosity, and the like. Further, conventional magnesium hydroxide has problems that it decreases the Izod impact strength of a resin when it is used as a neutralizing agent for an acid in the resin, that it causes a large amount of fish-eye when it is formed into a film, that it has a high sedimentation rate when it is used as an antacid in a suspension preparation, that it feels rough on the tongue, and the like.

Summary of the Invention

It is an object of the present invention to provide a novel magnesium hydroxide having high activity and high dispersibility, and a process for the production thereof.

It is another object of the present invention to provide a novel magnesium hydroxide having a large BET specific surface area, i.e. a small crystallite paticle diameter and forming an aggregate consising of a small number of crystallites, and a process for the production thereof.

It is still another object of the present invention to provide a novel magnesium hydroxide having an excellent viscosity thickening effect as a viscosity thickening agent for a resin, and a process for the production thereof.

It is further another object of the present invention to provide a novel magnesium hydroxide capable of neutralizing an acid in a resin without damaging the physical property, appearance, etc., of the resin when it is mixed into the resin.

It is yet another object of the present invention to provide a novel magnesium hydroxide which difficultly sediments and feels good on the tongue when it is used as an antacid in a suspension preparation, and a process for the production thereof.

The present invention provides a magnesium hydroxide having a BET specific surface area of 20 to 50 $m^2/g$, preferably 20 to 40 $m^2/g$, a 50 percent diameter, as a secondary particle, of not more than 1.5 $\mu$m, preferably not more than 1.0 $\mu$m, and a 90 percent diameter, as a secondary particle, of not more than 4.0 $\mu$m, preferably not more than 3.0 $\mu$m, more preferably not more than 2.0 $\mu$m.

The present invention also provides a process for the production of magnesium hydroxide which comprises heating a basic magnesium chloride or nitrate represented by formula

$Mg(OH)_{2-x}A_x \cdot mH_2O$

wherein A represents Cl or $NO_3$, x is in the range of 0<x<0.2, and m represents a number of from 0 to 6, and having main spacings of 8.2Å, 4.1Å, 2.7Å, 2.0Å, 1.56Å and 1.54Å measured by X-ray diffraction, in reaction mother liquor of the basic magnesium chloride or nitrate under atmospheric pressure or a pressure lower than atmospheric pressure at a temperature between about 50°C and about 120°C.


Brief Description of the Drawings

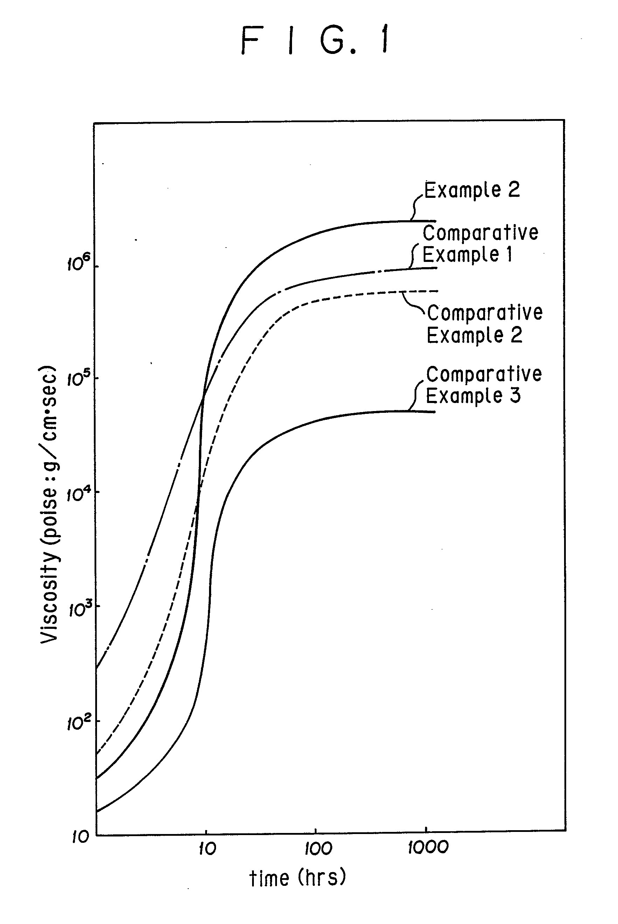

Figure 1 shows a difference in viscosity thickening effect when magnesium hydroxides obtained according to the present invention and a conventional process are incorporated into resins.

Detailed Description of the Invention

Conventional magnesium hydroxide has a 50 percent diameter of about 3 to 20 μm and a 90 percent diameter of about 8 to 40 μm even though it has a BET specific surface area in the range of from 20 to 50 $m^2/g$. In contrast, the magnesium hydroxide of the present invention is characterized in that it has, as a secondary particle, a much smaller particle diamter than conventional magnesium hydroxide, although it has a BET specific surface area overlapping with that of conventional magnesium hydroxide.

A magnesium hydroxide having a novel structure, which the present inventors have already proposed, has a strain, in the <101> direction in X-ray diffraction, of not more than $3.0 \times 10^{-3}$, a crystal particle diameter of over 800Å in the same direction, and a BET specific surface area of less than 20 $m^2/g$ (U.S. Patent 4,145,404). The magnesium hydroxide of the present invention differs in that it has a BET specific surface area of not less than 20 $m^2/g$ and a crystal particle diameter, in the <101> direction, of not less than 800Å. That is, although the above magnesium hydroxide having a novel structure has high dispersibility like the magnesium hydroxide of the present invention, its BET specific surface area is small. Namely, the above magnesium hydroxide having a novel structure has well-developed crystallinity and is chemically inactive. As opposed thereto, the magnesium hydroxide of the present invention has high dispersibility, and yet it has a greater BET specific surface area. That is, the magnesium hydroxide of the present invention is a very small crystal and is chemically active.

In general, with a decrease in the size of crystals, the crystals have a greater BET specific surface area and at the same time, has higher ability to aggregate, and the diameter of a secondary particle, i.e. an aggregate of crystals, increases. For this reason, it has been impossible to produce a high-dispersibility magnesium hydroxide having not only a BET specific surface area of not less than 20 $m^2/g$ but also a 50 percent diameter of not more than 1.5 μm and a 90 percent diameter of not more than 4.0 μm.

The magnesium hydroxide of the present invention can be produced by heating a basic magnesium chloride or nitrate represented by formula

$Mg(OH)_{2-x}A_x \cdot mH_2O$

wherein A represents Cl or $NO_3$, x is in the range of 0<x<0.2, and m represents a number of from 0 to 6, and having main spacings of 8.2Å, 4.1Å, 2.7Å, 2.0Å, 1.56Å and 1.54Å measured by X-ray diffraction, in reaction mother liquor of the basic magnesium chloride or nitrate under atmospheric pressure or a pressure lower than atmospheric pressure at a temperature between about 50°C and about 120°C.

The X-ray diffraction pattern of a basic magnesium chloride or nitrate usable in the present invention will be further detailed hereinbelow.

3

TABLE 1

| (basic magnesium chloride) | | |
|---|---|---|
| Spacing | Relative intensity | Face indices |
| $d_\text{Å}$ | $I/I_0$ | hkl |
| 8.2 | 100 | 003 |
| 4.1 | 48 | 006 |
| 2.70 | 27 | 101 |
| 2.26 | 34 | 106 |
| 2.03 | 9 | 108 |
| 1.56 | 39 | 110 |
| 1.54 | 14 | 113 |

TABLE 2

| (basic magnesium nitrate) | | |
|---|---|---|
| Spacing | Relative intensity | Face indices |
| $d_\text{Å}$ | $I/I_0$ | hkl |
| 8.2 | 100 | 003 |
| 4.1 | 42 | 006 |
| 2.65 | 15 | 102 |
| 2.37 | 15 | 105 |
| 2.02 | 9 | 108 |
| 1.56 | 19 | 110 |
| 1.54 | 9 | 113 |

The process for the production of a basic magnesium chloride or nitrate represented by the aforementioned formula and having a diffraction pattern detailed above is disclosed in U.S. Patent 4,145,404 issued to the present inventors. The summary of said process is as follows.

That is, said process comprises reacting magnesium chloride or magnesium nitrate with an alkali substance such as calcium hydroxide, ammonia, sodium hydroxide, potassium hydroxide, or the like in an aquesous medium, preferably in a a solution medium of calcium chloride in water, at a temperature of not more than about 40° C, preferably not more than 30° C, under a condition that an alkali to magnesium ions equivalent ratio is not more than 0.95, preferably not more than 0.9.

The high-activity high-dispersibility magnesium hydroxide of the present invention can be obtained by heating the above-produced basic magnesium chloride or nitrate in reaction mother liquor thereof under atmospheric pressure or a pressure lower than atmospheric pressure at a temperature between about 50° C and about 120° C. The co-presence of the reaction mother liquor is essential for formation of a high-activity high-dispersibility magnesium hydroxide forming an aggregate consisting of a small number of crystallites, since the reaction mother liquor works to prevent hydrolysis of the basic magnesium chloride or nitrate. It is preferable that calcium chloride is co-present in the reaction mother liquor. When calcium chloride is added to materials to be used for the formation of the basic magnesium chloride or nitrate, or when it is added to the basic magnesium chloride or nitrate before decomposition thereof under heat, the formed magnesium hydroxide has improved dispersibility. In order to achieve such an effect sufficiently, the necessary concentration of calcium chloride in the reaction mother liquor is at least 0.1 mole/ℓ, preferably 0.5 mole/ℓ.

The BET specific surface area tends to be smaller with an increase in a heating temperature, and the heating temperture may be hence changed to control the activity of the magnesium hydroxide as a product.

In addition, the process for the production of magnesium hydroxide, proposed by the present inventors in U.S. Patent 4,145,404, and the process for the production of a magnesium hydroxide in the present invention are distinguishable from each other in a condition for heating the basic magnesium chloride or nitrate and necessity for co-present reaction mother liquor. Due to differences mentioned here, the present invention makes it possible to produce a novel high-activity high-dispersibility magnesium hydroxide.

According to the present invention, there are provided a novel high-activity high-dispersibility magnesium hydroxide and a process for the production thereof.

According to the present invention, there are provided a novel magnesium hydroxide having a large BET specific surface area, i.e. a small crystal particle diameter and forming an aggregate consisting of a small number of crystallites, and a process for the production thereof.

According to the present invention there are provided a novel magnesium hydroxide having excellent viscosity thickening effect as a viscosity thickening agent for a resin, and a process for the production thereof.

According to the present invention there are provided a novel magnesium hydroxide capable of neutralizing an acid in a resin without damaging the physical property, appearance, etc., of the resin when it is mixed into the resin.

According to the present invention, there are provided a novel magnesium hydroxide which difficultly sediments and feels good on the tongue when it is used as an antacid in a suspension, and a process for the production thereof.

In the present invention, the BET specific surface area, the 50 percent and 90 percent diameters of secondary particles, and the crystal particle diameter in the <101> direction were measured in the following methods.

BET specific surface area:-

Measured in three-point plotting method according to a nitrogen adsorption method. An $N_2$ molecule adsorption area was calculated as $16.2Å^2$. Samples for measurement were subjected to gas exhaustion treatment under vacuume at $100°C$ for 30 minutes, and isothermic lines of nitrogen adsorption were measured.

Secondary particle diameter:-

A sample (0.7 g) was charged into a 100 m$\ell$ beaker, 70 ml of deionized water was gradually added, and the mixture was well dispersed. Then, the mixture was subjected to dispersion treatment using an ultrasonic homogenizer for 3 minutes. Immediately thereafter, part of the dispersion was taken, and the secondary particle diameter thereof was measured by using a microtrack particle size distrubution analyzer manufactured by Leeds & Northrop Instruments.

Crystal particle diameter in the <101> direction:-

Measured on the basis of peak profiles on (101) and (202) planes according to Jones' method (Jones, F. W., 1938, The measurement of particle size by the X-ray method; Rey, Sec. London A 166, 16-42).

The present invention will be illustrated by referring to Examples.

EXAMPLES 1~3

2 $\ell$ of a solution of 4.0 moles/$\ell$ of magnesium chloride in water (solution temperature $20°C$) was charged into a circular reactor of stainless steel having a volume of about 10 $\ell$, and stirred with a chemistirrer. 3.2 $\ell$, corresponding to an equivalent of 0.8 to magnesium, of a solution of 2 moles/$\ell$ of calcium hydroxide in water (solution temperature $20°C$) was all added thereto over about 2 minutes. Part of the resultant suspension containig a white precipitate was immediately filtered under reduced pressure, and the resultant substance was washed with a large amount of acetone, and dehydrated. The resultant product was dried for about 1 hour and subjected to X-ray diffraction and chemical analysis.

In the X-ray diffraction, the product showed the substantially same diffraction pattern as that of a basic magnesium chloride shown in Table 1. Further, the chemical analysis showed that the product had a composition of Mg(OH) $_{1.86}$Cl$_{0.14}$·mH$_2$O.

The suspension containing a white precipitate was separated into three portions, and these portions (containing reaction mother liquor and a precipitate each) were heated, without washing the precipitate with water, at 80°C for 2 hours (Example 1), at 90°C for 2 hours (Examples 2) and at 108°C for 2 hours (Examples 3). After the heat treatment, the resultant products were dehydrated, washed with water, dried and pulverized. In the pulverization, the products were pulverized by using an atomizer (manufactured by Fuji Powder K.K.) under a condition that the opening diameter of a screen was 2 mm, and then passed through a 200 mesh sieve. In the other Examples hereinbelow, the pulverizations were also carried out in the same way as in these particles.

X-ray diffraction patterns of the products showed that all the products were magnesium hydroxide. The BET specific surface areas, 50 percent diameters and 90 percent diameters of these products were measured, and Table 3 shows the results.

COMPARATIVE EXAMPLES 1~2

2 ℓ of a solution of 4.0 moles/ℓ of magnesium chloride in water (solution temperature 30°C) and 4.0 ℓ, corresponding to an alkali equivalent of 1.0 to magnesium, of a solution of 2 moles/ℓ of calcium hydroxide in water (solution temperature 30°C) were reacted by continuously charging them, at respective rates of 100 mℓ/minutes and 200 mℓ/minute, into a 10 ℓ reactor containing 2 ℓ of water at 30°C under stirring. The resultant reaction product was filtered under reduced pressure and the resultant substance was fully washed.

X-ray diffraction of the washed substance showed a diffraction pattern of magnesium hydroxide. Part of the washed substance was dehydrated, dried, pulverized and used for measurement (Comparative Example 1).

Another part of the washed substance was resuspended in water. Then, the suspension was heated and pressurized in an autoclave at 120°C for 4 hours, and the resultant substance was dehydrated, dried, pulverized, and used for measurement (Comparative Example 2).

Table 3 shows the BET specific surface areas, 50 percent diameters and 90 percent diameters of these products.

COMPARATIVE EXAMPLES 3

A reaction product obtained by repeating Example 1 was charged into an autoclave together with reaction mother liquor without washing it with water, and heated and pressurized at 150°C for 4 hours. The resultant substance was dehydrated, dried, pulverized, and used for measurement. Table 3 shows the BET specific surface area, 50 percent diameter and 90 percent diameter of the resultant magnesium hydroxide.

EXAMPLE 4

A 10 ℓ-capacity reactor containing 5 ℓ of a water solution of 2 moles/ℓ of magnesium chloride and 1.2 moles/ℓ of calcium chloride at a solution temperature of 15°C was charged with 4.5 ℓ of a solution of 4 moles/ℓ of ammonia in water (solution temperature 15°C), corresponding to an equivalent of 0.9 to magnesium, over about 5 minutes while the mixture was stirred with a chemistirrer. Part of a reaction product was taken, dehydrated, then washed with acetone, and dried at room temperature for 2 hours. The resultant product had a X-ray diffraction pattern nearly identical with that shown in Table 1. And chemical analysis of the product showed that it had a chemcial composition of Mg(OH)$_{1.82}$Cl$_{0.18}$mH$_2$O.

The remaining reaction product was heated at 70°C together with reaction mother liquor for 1 hour. The resultant substance was washed with water, dried, pulverized and used for measurement. Table 3 shows the BET specific surface area, 50 percent diameter and 90 percent diameter of the resultant product.

EXAMPLE 5

A 10 ℓ-capacity reactor containing 5 ℓ of a solution of 2 moles/ℓ of magnesium nitrate in water (solution temperature 25°C) was charged with 3.5 ℓ of a solution of 2 moles/ℓ of calcium hydroxide in water (solution temperature 25°C, corresponding to an equivalent of 0.7 to magnesium, over about 5 minutes while the mixture was stirred.

The resultant product had a X-ray diffraction pattern nearly identical with that shown in Table 2. And the chemical analysis of the product showed that it had a chemcial composition of $Mg(OH)_{1.88}(NO_3)_{0.12}mH_2O$.

The remaining reaction product was heated at 100°C together with reaction mother liquor for 1 hour. The resultant substance was washed with water, dried, pulverized and used for measurement. Table 3 shows the BET specific surface area, 50 percent diameter and 90 percent diameter of the resultant product.

## TABLE 3

| Example | BET specific surface area $(m^2/g)$ | Secondary particle diameter $(\mu m)$, 50 percent |
|---|---|---|
| 1 | 32 | 0.98 |
| 2 | 26 | 0.87 |
| 3 | 22 | 0.81 |
| 4 | 40 | 0.73 |
| 5 | 21 | 0.58 |
| Comparative Example | | |
| 1 | 38 | 4.17 |
| 2 | 22 | 2.90 |
| 3 | 7 | 0.78 |

## TABLE 3 (continued)

| Example | Secondary particle diameter $(\mu m)$, 90 percent | Crystal particle diameter in the $\langle 101 \rangle$ direction $(\text{Å})$ |
|---|---|---|
| 1 | 2.72 | 590 |
| 2 | 2.00 | 651 |
| 3 | 1.72 | 720 |
| 4 | 1.51 | 495 |
| 5 | 1.36 | 764 |
| Comparative Example | | |
| 1 | 17.21 | 367 |
| 2 | 9.87 | 518 |
| 3 | 1.61 | 1,825 |

REFERENTIAL EXAMPLE

100 parts by weight of an unsaturated polyester resin (trade name: Epolac, manufactured by Nippon Shokubai K.K.), 100 parts by weight of calcium carbonate (NS-100, made by Nitto Funka K.K.), and 4 parts by weight of magnesium hydroxide (viscosity thickening agent) were mixed in a homogenizere for 2 minutes, and the mixture was put in an oven at 35°C to measure change of viscosity with time by using a Brookfield viscometer. Magnesium hydroxides obtained in Example 2, and Comparative Examples 1 to 3 were used for the measurement. Figure 1 shows the results.

**Claims**

1. A magnesium hydroxide having a BET specific surface area of 20 to 50 m²/g, a 50 percent diameter, as a secondary particle, of not more than 1.5 $\mu$m and a 90 percent diameter, as a secondary particle, of not more than 4.0 $\mu$m.

2. A magnesium hydroxide according to claim 1 wherein the BET specific surface area is 20 to 40 m²/g.

3. A magnesium hydroxide according to claim 1 or 2 wherein the 50 percent diameter, as a secondary particle diameter, is not more than 1.0 $\mu$m.

4. A magnesium hydroxide according to claim 1, 2 or 3 wherein the 90 percent diameter, as a secondary particle diameter, is not more than 3.0 $\mu$m.

5. A process for the production of a magnesium hydroxide which comprises heating a basic magnesium chloride or nitrate of the formula $Mg(OH)_{2-x}A_x.mH_2O$ wherein A represents Cl or $NO_3$, x is in the range of $0<x<0.2$, and m represents a number of from 0 to 6, and having main spacings of 8.2Å, 4.1Å, 2.7Å, 2.0Å, 1.56Å and 1.54Å measured by X-ray diffraction, in a reaction mother liquor of the basic magnesium chloride or nitrate under atmospheric pressure or below at a temperature between 50°C and 120°C.

6. A process according to claim 5 which uses a basic magnesium chloride or nitrate obtained by reacting magnesium chloride or magnesium nitrate with an alkali substance in an aqueous medium at a temperature of not more than 40°C and under a condition that the alkali to magnesium ions equivalent ratio is not more than 0.95/1.

7. A process according to claim 5 or 6 wherein the reaction mother liquor contains at least 0.1 mole/ of calcium chloride.

# F I G. 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 104, no. 4, January 1986 Columbus, Ohio, USA page 108; right-hand column; ref. no. 21395 P &JP-A-60176918(KIYOUWA KAGAKU KOGYO) * abstract * | 5-7 | C01F5/14        2 C01F5/20 C01F5/22 |
| A | EP-A-189098 (ASAHI GLASS) * claims 1, 2 * | 1-4 | |
| A | EP-A-69961 (KYOWA CHEM IND) * page 6, lines 14 - 24 * | 1-4 | |
| A | * claims 4, 6 * | 5-7 | |
| D,A | US-A-4145404 (MIYATA) | | |

TECHNICAL FIELDS
SEARCHED (Int. Cl.5)

C01F
C04B

The present search report has been drawn up for all claims

| Place of search | Date of completion of the search | Examiner |
|---|---|---|
| THE HAGUE | 29 JANUARY 1990 | ZALM W. E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 
& : member of the same patent family, corresponding document

EPO FORM 1503 03.82 (P0401)